# EUROPÄISCHE PATENTSCHRIFT

(11) **EP 3 242 633 B1**
(45) Veröffentlichungstag und Bekanntmachung des Hinweises auf die Patenterteilung: **26.03.2025**
(21) Anmeldenummer: 15808221.4
(22) Anmeldetag: 11.12.2015
(51) Int. Cl.: A61F 2/32

(54) **DOUBLE MOBILITY PROTHESE**
DOUBLE MOBILITY PROSTHESIS
PROTHÈSE À DOUBLE MOBILITÉ

(30) Priorität: 07.01.2015 DE 202015100029 U
(43) Veröffentlichungstag der Anmeldung: 15.11.2017
(73) Patentinhaber: Waldemar Link GmbH & Co. KG, 22339 Hamburg (DE)
(72) Erfinder: LINK, Helmut D., 22397 Hamburg (DE); JENDRO, Günther, 24568 Kaltenkirchen (DE); GRADL, Georg, 85598 Baldham (DE)
(74) Vertreter: Alatis
(86) Internationale Anmeldenummer: PCT/EP2015/079419
(87) Internationale Veröffentlichungsnummer: WO 2016/110372

(56) Entgegenhaltungen:
- EP-A1- 1 776 934
- EP-A1- 2 489 333
- WO-A1-02/00141
- WO-A2-2006/084426
- FR-A1- 2 795 302
- FR-A1- 2 948 013
- US-A- 4 642 123
- US-A1- 2014 128 988

## Beschreibung

### GEBIET DER ERFINDUNG

Die vorliegende Erfindung betrifft einen Gelenkersatz mit zwei Teilgelenken sowie eine Einsetzhilfe für diesen Gelenkersatz.

### STAND DER TECHNIK

Das Ersetzen eines verletzten oder abgenutzten natürlichen Gelenks mit einem künstlichen Gelenk ist ein alltäglicher Eingriff. So gibt es mittlerweile künstlichen Gelenkersatz für eine Vielzahl von natürlichen Gelenken, wie zum Beispiel für das Hüftgelenk, das Schultergelenk, das Ellenbogengelenk, das Sprunggelenk, das Fingergelenk oder das Kniegelenk. Ein vollständiger bzw. totaler Gelenkersatz besteht dabei aus mindestens zwei Komponenten, die an den sich gegenüberliegenden Knochen fixiert werden. So weisen deartige vollständige Hüftendoprothesen eine Gelenkpfanne auf, die im Acetabulum eines Patienten implantiert wird, sowie einen Gelenkkopf auf einem Prothesenschaft zur Verankerung im Femur.

Im Allgemeinen werden künstliche Gelenke in Bezug auf ihre Abmessungen und ihren Aufbau nicht einfach den natürlichen Gelenken nachgeahmt. So sind zum Beispiel bei künstlichen Hüftgelenken im Verhältnis zum natürlichen Gelenkkopf die Gelenkköpfe häufig mit einem wesentlich kleineren Durchmesser versehen. Gründe hierfür sind ein verminderter Abrieb oder der Einsatz von Keramik als Prothesenwerkstoff. Bei dem Einsatz einer Keramik haben kleinere Köpfe aufgrund ihres kleineren Volumens den Vorteil, dass das Risiko eines Versagens durch herstellungsbedingte Fehler reduziert wird. Weiter verursachen Gelenkköpfe mit einem kleineren Durchmesser aufgrund der geringeren Bewegung im Gelenkspalt einen geringeren Abrieb. Dagegen haben Gelenkköpfe mit größerem Durchmesser den Vorteil, dass sie eine höhere Stabilität sowie ein geringeres Luxationsrisiko aufweisen und dem Patienten einen größeren Bewegungsumfang bieten.

Doppelgelenkige Prothesen versuchen, diese sich in Bezug auf den größeren Gelenkkopfdurchmesser ergebenden Vorteile in einer Prothese mit einem kleinen Gelenkkopf umzusetzen. Derartige Hüftendoprothesen umfassen im Regelfall drei Komponenten, die relativ zueinander beweglich sind. Im Vergleich zu den oben genannten Standardprothesen befindet sich zwischen der Hüftpfanne und dem Hüftkopf ein Gelenkeinsatz, der sich relativ zu der Hüftpfanne und dem Hüftkopf bewegen kann. Folglich kombiniert eine solche Hüftendoprothese im Prinzip eine Gelenkprothese mit großem Durchmesser und eine Gelenkprothese mit kleinem Durchmesser, im Folgenden auch als erstes bzw. großes Teilgelenk und zweites bzw. kleines Teilgelenk bezeichnet.

Das große Gelenk wird von der konkaven Fläche der Hüftpfanne und der konvexen Fläche des Gelenkeinsatzes ausgebildet, während das kleine Gelenk aus der konkaven Fläche des Gelenkeinsatzes und der konvexen Fläche des Hüftkopfes, gebildet wird, der üblicherweise mit einem in einem Femur verankerten Prothesenschaft verbunden ist.

Dieser mehrgelenkige Aufbau hat zur Folge, dass das Bewegungsausmaß auf das große Gelenk und das kleine Gelenk aufgeteilt wird. Dabei führt das kleine Gelenk vor allem Bewegungen aus, die nur einen kleinen Bewegungsumfang erfordern, wie zum Beispiel der normale Gang. Erreicht das kleine Gelenk allerdings seinen maximalen Ausschlag, so wird spätestens dann auch das große Gelenk ausgelenkt und somit der mögliche Bewegungsumfang der gesamten Hüftgelenkendoprothese ausgenutzt.

Bei so einem Bewegungsablauf wird also einerseits das Bewegungsausmaß des großen Gelenks minimiert, während die Vorteile eines großen Bewegungsumfangs und eines geringeren Luxationsrisikos beibehalten werden. Andererseits findet bevorzugt ein Großteil der Gelenkbewegung zwischen den Laufflächen des kleinen Gelenks statt, was zu einem insgesamt geringen Verschleiß der Hüftendoprothese beiträgt.

Damit allerdings ein Zusammenhalt derartig aufgebauter Hüftendoprothesen sichergestellt ist, wird eine der gelenkigen Verbindungen als Nussgelenk ausgeführt. Ein solches Nussgelenk zeichnet sich dadurch aus, dass die konvexe Gelenkfläche des Gelenkkopfes über seinen Äquator hinaus von der konkaven Gelenkfläche der Gelenkpfanne eingefasst ist, sodass einem Auseinanderfallen dieser Komponenten vorgebeugt ist. Ein solches Nussgelenk sollte allerdings intraoperativ montierbar sein, da ein bereits montiertes Gelenk die Implantation erschwert und beim Einschlagen des Gelenks womöglich sogar Schäden auftreten könnten.

Um einen Gelenkkopf in die gegenüberliegende Gelenkpfanne einzusetzen, damit die konvexe Gelenkfläche mit der konkaven Gelenkfläche in Kontakt kommt, kann der Kopf in den Gelenkeinsatz einschnappbar ausgeführt sein. Hierfür ist allerdings ein gewisser Kraftaufwand notwendig, um den Gelenkkopf bzw. die Öffnung der Pfanne soweit zu verformen, dass der Kopf in der Pfanne zum Sitzen kommt.

Eine andere Möglichkeit der Montage eines solchen Gelenks wird von der US Patentanmeldung 2010/0234963 A1 vorgeschlagen, bei welcher die Öffnung der konkaven Fläche der Pfanne so ausgebildet ist, dass der Hüftkopf nur um 90° verdreht durch diese geführt werden kann. Einmal eingeführt, lässt sich der Hüftkopf wieder zurückdrehen, sodass die Pfanne und der Kopf zueinander ausgerichtet sind. Allerdings befindet sich der Rand des Hüftkopfes in einem derartig ausgestalteten Gelenksystem im eingesetzten Zustand zwangsläufig unterhalb der Pfannenöffnung. Damit hat eine solche Anordnung den Nachteil, dass sie eine geometrisch bedingte Einschränkung des Bewegungsumfangs nach sich zieht, da der mit dem Hüftkopf verbundene Implantatschaft früher an den Rand der Pfannenöffnung stößt.

Die Lehre dieses Dokuments beschäftigt sich dabei allerdings nicht mit dem altersbedingten Luxationsrisiko. So ist bei älteren Patienten das Luxationsrisiko neben der operationsbedingten Dehnung und Beschädigung ihres Gewebes zusätzlich erhöht, da es im Regelfall von vornherein geschwächt ist. Dies ist insbesondere dann der Fall, wenn der Implantation eines Gelenksersatzes eine längere Phase eingeschränkter Mobilität aufgrund des verbrauchten Gelenks vorausgegangen ist. Zwar kann diese Situation durch physiotherapeutische Maßnahmen präoperativ verbessert werden, jedoch ist es wünschenswert, weitere Behandlungsmöglichkeiten für derartige Patienten zur Verfügung zu haben, welche die begrenzten Möglichkeiten dieser physiotherapeutischen Maßnahmen ergänzen können.

Aus der WO 02/00141 A1 ist eine Gelenkprothese bekannt, die mindestens ein festes Element, das dazu bestimmt ist, mit einem Organ des menschlichen Körpers, insbesondere einem Knochen, fest verbunden zu werden, mindestens ein bewegliches Element, das sich in Bezug auf das feste Element bewegen kann, sowie mindestens ein Zwischenelement umfasst, das die Gelenkigkeit des beweglichen Elements in Bezug auf das feste Element ermöglicht. Das Zwischenelement wird in einem Innenvolumen des festen Elements aufgenommen, wobei dieses Zwischenelement mindestens einen Rotationsfreiheitsgrad in Bezug auf dieses feste Element besitzt, während das bewegliche Element zumindest teilweise in einem Innenvolumen des Zwischenelements aufgenommen wird und mindestens einen Rotationsfreiheitsgrad in Bezug auf dieses Zwischenelement besitzt. Es sind Mittel vorgesehen, die die translatorische Verbindung des beweglichen Elements in Bezug auf das Zwischenelement ermöglichen.

Aus der US 2014/128988 sind prothetische Hüftköpfe, bewegliche Einsätze und Hüftpfannenkomponenten vorbekannt, die die Beeinträchtigung von Weichgewebe verringern, den Implantatverschleiß reduzieren und/oder das Reibungsdrehmoment verringern können. Es werden modulare Verbindungen bereitgestellt, die das Auftreten von Lockerungen und Mikrobewegungen minimieren können, die an modularen Verbindungen von orthopädischen Implantaten auftreten können.

### ZUSAMMENFASSUNG DER ERFINDUNG

Aufgabe der vorliegenden Erfindung war es somit, einen Einsatz und ein Gelenksystem bereitzustellen, das auch bei Patienten mit eingeschränkter Konstitution eine Luxation verhindert. Eine weitere Aufgabe war es, eine einfache intraoperative Montage zu ermöglichen, ohne dabei die Funktionalität der Gelenkendoprothese einzuschränken. Dabei sollte die Belastung der Gelenkkomponenten möglichst niedrig gehalten werden.

Diese Aufgabe wird durch die in den unabhängigen Ansprüchen definierte Kombination von Merkmalen gelöst. Die zugehörigen abhängigen Ansprüche beschreiben dabei bevorzugte Ausführungsformen der vorliegenden Erfindung.

Zur Lösung der Aufgabe stellt die vorliegende Erfindung einen Gelenkersatz gemäß Anspruch 1.

Die Sicherungseinrichtungen der Gelenkpfanne und des Gelenkeinsatzes ermöglichen es, das Luxationsrisiko insbesondere bei Patienten mit geschwächtem Gewebe zu minimieren oder sogar auszuschließen. Somit können auch Patienten einen Gelenkersatz erhalten, bei denen ein solcher aufgrund des Gewebezustands zuvor nur schwer oder nicht möglich war. Die damit wiedergewonnene Bewegungsfreiheit bedeutet für diese Patienten einen erheblichen Zugewinn an Lebensqualität.

Bei einer besonders bevorzugten Ausführungsform der vorliegenden Erfindung, beugt ein Bereich der konkaven Gelenkfläche der Gelenkpfanne und/oder des Gelenkeinsatzes der Luxation des jeweiligen Teilgelenks durch Ausbilden des jeweiligen Gelenks als Nussgelenk vor.

Die Sicherungseinrichtungen des erfindungsgemäßen Gelenkersatzes sind also so ausgeführt, dass die Teilgelenke auch ohne äußere Krafteinwirkung im montierten Zustand verbleiben, ohne dass dabei ihre Bewegungsfreiheit eingeschränkt wird.

Der Zusammenhalt der Gelenkkomponenten wird dadurch gewährleistet, dass die Einsatzöffnung des aufnehmenden Gelenkteils kleiner ist als die Außenabmessung des einzusetzenden Gelenkteils. Anders ausgedrückt ist die Einsatzöffnung der konkaven Gelenkfläche so ausgebildet, dass sie kleiner ist als der größte Abstand zwischen zwei Punkten auf der konvexen Fläche des Einsatzes, zumindest wenn es sich dabei um ein Kugelgelenk handelt. Folglich befindet sich der Bereich, welcher einer Luxation vorbeugt, bei der umlaufenden Kante der konkaven Gelenkfläche. Unter der Annahme einer Kugelform des Gelenks bildet der Bereich somit den Teil der Gelenkfläche aus, der über deren Äquator hinausgeht.

Bei einer weiteren bevorzugten Ausführungsform ist der Öffnungsdurchmesser der konkaven Gelenkfläche 1% bis 6%, bevorzugt 2% bis 5%, am bevorzugtesten 3,5% bis 5% geringer als der Gelenkdurchmesser.

Hierdurch kann einer Luxation des im Patienten implantierten Gelenks effektiv vorgebeugt bzw. diese verhindert werden. Dabei ist es insbesondere bei den unteren Werten dieser Bereiche möglich, die Gelenkpartner durch Einpressen des männlichen Gelenkteils in das weibliche Gelenkteil zu montieren. Dabei sollte jedoch zumindest einer der Gelenkpartner möglichst aus einem Polymer, insbesondere UHMWPE, hergestellt sein. Dabei kann das Einpressen vor der Implantation des Gelenkersatzes erfolgen, um eine Beschädigung des Patientengewebes zu vermeiden.

Bei einer besonders bevorzugten Ausführungsform weist die Sicherungseinrichtung von zumindest einem der Teilgelenke zwei bevorzugt lösbare Sicherungselemente auf, die zusammenwirkend einer Luxation des Teilgelenks vorbeugen.

Anders als bei der oben beschriebenen Montage des Gelenks durch Zusammenpressen kann die Montage des Gelenks mithilfe der Sicherungselemente auch bei der Implantation erfolgen. Mit anderen Worten ist bei dem erfindungsgemäßen Gelenkersatz bevorzugt nur eines der Teilgelenke als Nussgelenk ausgeführt, das durch Zusammenpressen der beiden Gelenkkomponenten montiert wird. Zumindest das andere Teilgelenk sollte bei dieser Ausführungsform hingegen, insbesondere wenn es als Nussgelenk ausgebildet ist, mit Hilfe von lösbaren Sicherungselementen montierbar sein. Diese ermöglichen, den oben erwähnte Bereich der konkaven Gelenkfläche, welcher das Gelenk als Nussgelenk ausbildet, erst nach dem Einsetzen des konvexen Gelenkpartners mit der konkaven Gelenkfläche zu verbinden.

Dass zumindest eines der Teilgelenke mit einer lösbare Sicherungselemente aufweisenden Sicherungseinrichtung ausgebildet ist, hat zudem im Falle einer Reversion Vorteile, da hierdurch eine Entnahme der auszutauschenden Gelenkkomponenten vereinfacht wird.

Bei einer weiteren besonders bevorzugten Ausführungsform wirken die zwei Sicherungselemente mittels einer bevorzugt lösbaren Schraub-, Verriegelungs- und/oder Schnappverbindung zusammen.

Die so ausgeführte Sicherungseinrichtung ist einfach montierbar und kann zudem auch lösbar ausgeführt sein.

Beispielsweise kann die Sicherungseinrichtung in Form einer Schraubverbindung ausgebildet sein, da diese einfach montierbar und auch leicht wieder lösbar ist. Um einem ungewollten Lösen der Sicherungseinrichtung dieser Ausführungsform vorzubeugen, ist diese bevorzugt über einen Form- oder Reibschluss mit einem Blockierelement gesichert.

Bei einer weiteren Ausführungsform der vorliegenden Erfindung ist ein Sicherungselement durch eine bevorzugt umlaufende Aussparung zur Aufnahme eines Sicherungsrings in der konkaven Gelenkfläche der Gelenkpfanne und/oder des Gelenkeinsatzes ausgebildet.

Bei dieser Ausführungsform kann aufgrund der Verwendung eines Sicherungsrings bei der Montage jegliche Belastung auf das Gewebe vermieden werden. Ähnliches kann auch für eine Schnappverbindung gelten, die über einen Sicherungsring gesichert wird.

Als Sicherungsring wird bevorzugt ein länglicher Streifen aus einem flexiblen Material verwendet, der noch bevorzugter als C-förmiger Ring vorgeformt ist. Der Streifen ist über eine Zugangsöffnung oder -aussparung, die sich vom Rand der konkaven Gelenkfläche bis zu der umlaufenden Aussparung erstreckt, der Länge nach in die Aussparung einführbar.

Bei einer weiteren erfindungsgemäßen Ausführungsform ist ein Sicherungselement durch mindestens eine Aussparung in der Gelenkpfanne und/oder in dem Gelenkeinsatz ausgebildet, welches mit einem Vorsprung eines weiteren Sicherungselements als Bajonettverbindung wirkt.

Wie bereits oben im Zusammenhang mit dem Gewinde beschrieben, ist auch diese Sicherungseinrichtung einfach montierbar und lösbar. Der Vorteil dieser Ausführungsform liegt darin, dass mit einem Bajonettverschluss die Verriegelung der Sicherheitselemente sowohl durch Ausübung einer Kraft wie auch eines Moments erfolgt und so die Belastung des Gewebes beispielweise gegenüber einem reinen Moment zur Montage des Gelenks verringert werden kann.

Die Einsetzhilfe kann selbstverständlich bei dem ersten und/oder dem zweiten Teilgelenk zum Einsatz kommen, d. h. auch oder alternativ zwischen dem Gelenkeinsatz und der konvexen Fläche des Gelenkkopfes.

Die Einsetzhilfe hat insbesondere bei einer Ausbildung eines Teilgelenks als Nussgelenk, das ohne Einsetzhilfe nur durch Zusammenpressen der Gelenkteile vor der Implantation zusammengesetzt werden sollte, den Vorteil, dass die Einpresskraft vermindert oder sogar vermieden wird. Gleichzeitig wird eine Luxation des Gelenks im Bereich des möglichen Bewegungsumfangs des implantierten Gelenks verhindert. Folglich kann mit Hilfe einer Einsetzhilfe ein Teilgelenk intraoperativ montiert werden, das ansonsten nur vor der Implantation zusammengesetzt werden könnte. Hierdurch kann das Luxationsrisiko bei den im Alltag vorkommenden Belastungen ausgeschlossen werden.

Die zum Montieren des Teilgelenks notwendige Verformung wird dadurch vermindert oder umgangen, dass die Aussparung mit ihrer bevorzugt länglichen Form den Durchmesser der konvexen Gelenkfläche partiell reduziert. Dank der Einsetzhilfe ist der notwendige Kraftaufwand beim Einsetzen in die konkave Gelenkfläche des Nussgelenks, wenn überhaupt vorhanden, so gering, dass insbesondere intraoperativ die Belastung für das Gewebe des Patienten reduziert sowie einer Beschädigung der Gelenkkomponenten vorgebeugt wird.

Die auf der Gleitfläche angeordnete längliche Aussparung der Einsetzhilfe ermöglicht ein erleichtertes Einsetzen der konvexen Gelenkfläche in die konkave Gelenkfläche des jeweiligen Teilgelenks. Je nach Dimensionierung der Aussparung kann dieses Einsetzen ohne jegliche Verformung oder mit einer Verformung ausgeführt werden, die lediglich Spannungen verursacht, bei denen keine Beschädigungen der Materialien der Gelenkpfanne bzw. des Einsatzes auftreten. Folglich ist es mit diesem Einsatz möglich, einen Überdeckungsgrad des Nussgelenks zu erreichen, der höher ist als der, welcher durch ein Einsetzen mittels einer Schnappverbindung erreicht werden kann. Zudem beugt die Aussparung beim Einsetzen einem unkontrollierten Wegrutschen bei der Montage vor, wie es beispielsweise bei einer Schnappverbindung auftreten kann.

Die längliche Form der Einsetzhilfe auf der konvexen Gelenkfläche hat insbesondere den Vorteil, dass die Einsetzhilfe so der Kontur der Öffnungskante der konkaven Gelenkfläche angepasst werden kann. Zudem wird auf diese Weise sichergestellt, dass die Einsetzhilfe aufgrund ihrer minimalen Größe einen möglichst geringen Einfluss auf die Geometrie der konvexen Fläche des Einsatzes aufweist.

Für das Einsetzen der konvexen Gelenkkomponente mit Hilfe der Einsetzhilfe werden die beiden Gelenkpartner relativ zueinander geneigt. Um eine Luxation zu verursachen, müsste folglich das Gelenk zum gleichen Ausmaß wie beim Einsetzen ausgelenkt werden. Durch das Platzieren der Einsetzhilfe in einer Symmetrieebene der konvexen Gelenkfläche wird unabhängig von der Ausrichtung des Gelenks nach Implantation erreicht, dass das Teilgelenk um nahezu 90° ausgelenkt werden müsste, um eine Luxation zu verursachen. Eine derartige Auslenkung ist, insbesondere beim Hüftgelenk, physiologisch im Regelfall weder physiologisch noch aufgrund des Prothesenhalses erreichbar.

Eine noch höhere Absicherung gegen eine Luxation ist möglich, wenn die Ausrichtung der Einsetzhilfe im implantierten Zustand berücksichtigt wird. Mit anderen Worten wird die Einsetzhilfe so auf der konvexen Gelenkfläche des Teilgelenks positioniert, dass sie nach der Implantation durch den physiologischen Bewegungsumfang nicht erreicht werden kann. Beispielsweise ist es von Vorteil, bei einem Hüftgelenk die Einsetzhilfe hinter dem Bereich der maximalen Adduktion zu platzieren.

Auch wenn die Einsetzhilfe vor allem bei einem Doppelgelenk von Vorteil ist, um einer Luxation vorzubeugen, können die oben genannten Vorteile mit den entsprechenden Merkmalen auch bei einem einfachen Nussgelenk erreicht werden.

### KURZE BESCHREIBUNG DER FIGUREN

Mit Hilfe der folgenden Figuren und Beschreibung werden Ausführungsbeispiele für ein besseres Verständnis der vorliegenden Erfindung im Detail beschrieben. Hierfür wurden die in den Figuren ersichtlichen Merkmale mit Bezugszeichen gekennzeichnet. Dabei wurden bei unterschiedlichen Ausführungsbeispiele gleiche Bezugszeichen verwendet, sofern sich die Merkmale in diesen Ausführungsbeispielen gleichen oder eine gleiche Wirkung erzielen.
Figur 1 zeigt einen erfindungsgemäßen montierten Gelenkersatz, der zwei Teilgelenke aufweist.
Figur 2 zeigt einen erfindungsgemäßen als Nussgelenk ausgeführten Gelenkeinsatz während des Einsetzens eines an einem Prothesenschaft montierten Gelenkkopfes.
Die Figuren 3a und 3b zeigen einen erfindungsgemäßen Einsatz, der auf seiner konvexen Gelenkfläche eine Einsetzhilfe aufweist.
Die Figuren 4a und 4b zeigen eine erfindungsgemäße implantierbare Gelenkpfanne.
Die Figuren 5a und 5b zeigen das Einsetzen eines erfindungsgemäßen Gelenkeinsatzes mittels einer länglich ausgeführten Einsetzhilfe.

### AUSFÜHRLICHE BESCHREIBUNG BEVORZUGTER AUSFÜHRUNGSFORMEN

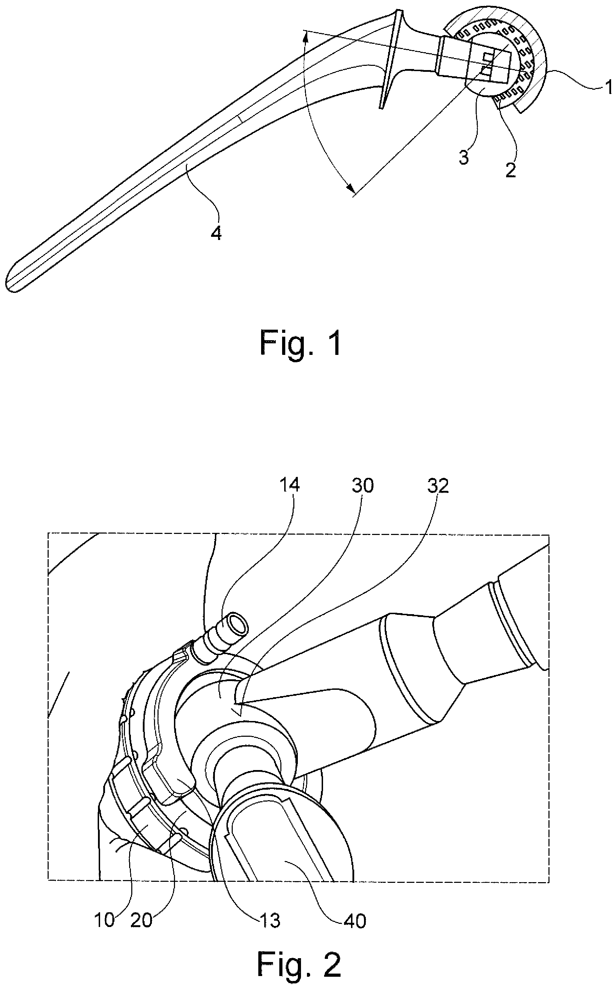

Figur 1 zeigt den montierten Zustand einer doppelgelenkigen Hüftendoprothese, die auch als Double Mobility Prothese bezeichnet wird. Derartige Prothesen aus dem Stand der Technik ermöglichen, wie oben bereits beschrieben, trotz eines kleinen Hüftkopfes 3 den Bewegungsumfang einer Großkopfprothese. Das in Figur 1 in der Abduktionsstellung gezeigte Implantat kombiniert dabei die Vorteile geringeren Abriebs eines kleineren Hüftkopfes mit dem größeren Bewegungsumfang einer Großkopfprothese, indem es über zwei Teilgelenke aufgebaut ist.

Das erste Teilgelenk befindet sich zwischen der im Beckenknochen implantierten Hüftpfanne 1 und einem Gelenkeinsatz 2. Das zweite Teilgelenk wird zwischen dem Gelenkeinsatz 2 und dem Hüftkopf 3 ausgebildet. Wie durch die Auslenkung des Implantats mit maximalem Abduktionswinkel in Figur 1 erkennbar, vergrößert sich der Bewegungsumfang dadurch, dass der unter dem Hüftkopf 3 befindliche Prothesenschaft 4 zwar an den Rand des Gelenkeinsatzes 2 stößt, dieser jedoch durch die Beweglichkeit des ersten Teilgelenks ausgelenkt wird, sodass der Bewegungsumfang bis zum Anschlag des Prothesenschaft 4 an den inneren Rand der Hüftpfanne 3 vergrößert wird.

Bei dem in Figur 1 gezeigten Implantat weist das erste Teilgelenk zwischen der Hüftpfanne 1 und dem Gelenkeinsatz 2 Gelenkkomponenten eines Kugelgelenks auf, die auseinanderfallen, wenn sie nicht durch äußere Kräfte zusammengehalten werden. Hingegen handelt es sich bei dem zweiten Teilgelenk zwischen dem Gelenkeinsatz 2 und dem Hüftkopf 3 um ein Nussgelenk. Bei diesem Gelenk umschließt der Gelenkeinsatz 2 den Hüftkopf 3 über dessen Äquator hinaus und ist folglich so an diesem montiert, dass die beiden Gelenkkomponenten des zweiten Teilgelenks auch ohne äußere stabilisierende Krafteinwirkung nicht auseinanderfallen.

Figur 2 zeigt eine erfindungsgemäße Hüftendoprothese beim Einsetzen des Hüftkopfes 30 in den Gelenkeinsatz 20. Dabei wird der mit dem Prothesenschaft 40 verbundene Gelenkkopf 30 mithilfe eines Drückwerkzeugs 50 in die konkave Gelenkfläche 21 des Gelenkeinsatzes 20 eingesetzt. Eine Gelenkblockiereinrichtung 13 stellt beim Einpressen des Gelenkkopfes 30 über die umlaufende Kante 23 der konkaven Fläche 21 des Gelenkeinsatzes 20 sicher, dass der Gelenkeinsatz 20 während der Verwendung des Werkzeugs 50 nicht relativ zu der Gelenkpfanne 10 verschwenken kann. Dazu ist die Gelenkblockiereinrichtung 13 wie weiter unten beschrieben über ein Verbindungselement 14 an dem Rand 15 der Gelenkpfanne 10 gesichert, der sich zwischen der Außenfläche 17 der Gelenkpfanne 10 und der konkaven Innenfläche 11 der Gelenkpfanne befindet bzw. diese verbindet. So befestigt, erstreckt sich ein Abschnitt der Gelenkblockiereinrichtung über den Rand des Gelenkeinsatzes 20, der sich zwischen der umlaufenden Kante 23 der konkaven Gelenkfläche 21 und der umlaufenden Kante 24 der konvexen Gelenkfläche 22 befindet. Vorteilhafterweise erstreckt sich der Abschnitt der Gelenkblockiereinrichtung 13 dabei um über mehr als 180° des ringförmigen Rands des Gelenkeinsatzes 20, um ein Ausdrehen des Gelenkeinsatzes in allen Richtungen zu unterbinden.

Selbstverständlich ist es möglich, auch für den Gelenkkopf 30 eine derartige Gelenkblockiereinrichtung 13 vorzusehen, um das Einsetzen des Gelenkkopfes 30 in die konkave Gelenkfläche 21 des Gelenkeinsatzes 20 zu erleichtern.

Wenn die Montage eines oder beider Teilgelenke nach Implantation der Gelenkpfanne durchgeführt wird, kann die Gelenkblockiereinrichtung 13 zudem so ausgelegt sein, dass das Drückwerkzeug 50 diese als Widerlager verwendet. Bei so einer Ausführung stützt sich das Drückwerkzeug an der Blockiereinrichtung 13 ab, sodass bestenfalls keine Kraft auf das umliegende Knochengewebe ausgeübt wird.

Weiterhin ist in Figur 2 zu erkennen, dass der Rand des Gelenkeinsatzes 20 in seiner neutralen Position, d.h. die Rotationssymmetrieachse des Gelenkeinsatzes 20 und der Gelenkpfanne 10 liegen aufeinander, mit dem Rand 15 der Gelenkpfanne 20 fluchtet. Genauso ist es möglich, den umlaufenden Rand des Gelenkeinsatzes 20 in Bezug auf den Rand 15 der Gelenkpfanne 20 in neutraler Position bzw. Ausgangsposition hervorstehen zu lassen. Damit lässt sich bei gleichbleibendem Bewegungsumfang die Bewegungshäufigkeit zwischen der Gelenkpfanne 10 und dem Gelenkeinsatz 20 erhöhen. Anders gesagt, kommt es bei einer Auslenkung des Gelenkkopfes 30 früher zu einer Berührung des Prothesenschafts 40 mit dem umlaufenden Rand des Gelenkeinsatzes 20 und damit früher zu einer aufgezwungenen Relativbewegung zwischen dem Einsatz 20 und der Gelenkpfanne 10. Da dies, wie oben beschrieben, einen erhöhten Abrieb zur Folge haben kann, wird die in Figur 2 gezeigte Ausführungsform bevorzugt.

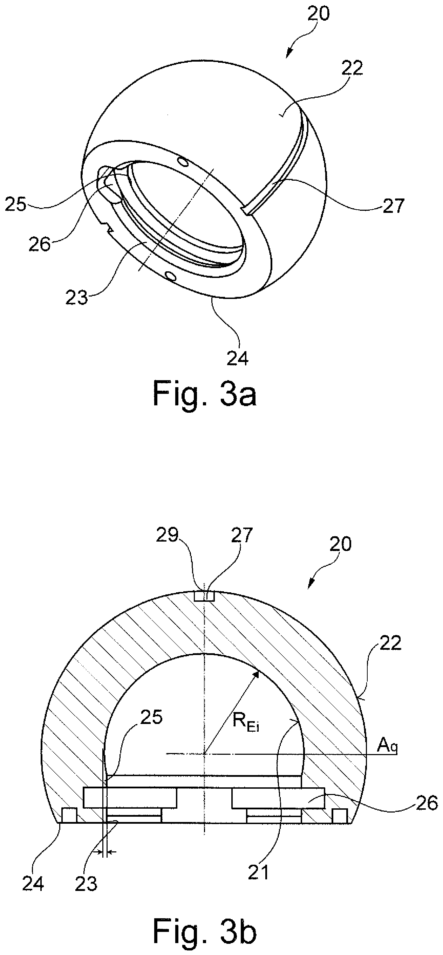

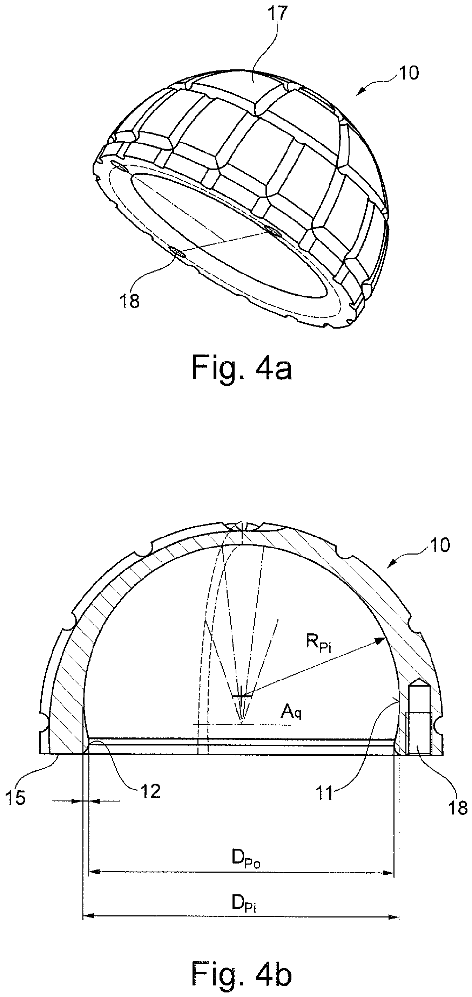

Die Figuren 3a und 3b zeigen einen erfindungsgemäßen Gelenkeinsatz 20. Wie in Figur 3b zu erkennen, ist die konkave Gelenkfläche 21 des Gelenkeinsatzes 20 als Nussgelenk ausgebildet. Gleiches gilt für die konkave Gelenkfläche 11 in der in Figur 4b gezeigten Schnittansicht der Gelenkpfanne 10. Folglich weist die konkave Aufnahme bei beiden Teilgelenken eine Einsetzöffnung auf, die kleiner ist als die in ihr aufzunehmende konvexe Gelenkfläche, sodass die Montage der Teilgelenke Kraftaufwand und/oder eine Einsetzhilfe 27 erfordert.

Da in dem in den Figuren 3 und 4 gezeigten Ausführungsbeispiel beide Teilgelenke kugelförmig ausgebildet sind, ist der Radius R_{Ei} der konkaven Gelenkfläche 21 größer als der Radius der Öffnung der durch die konkave Gelenkfläche 21 ausgebildeten Aufnahme bzw. ist der Durchmesser D_{Pi} der konkaven Gelenkfläche 11 der Gelenkpfanne 10 größer als der Durchmesser D_{Po} der Gelenkpfannenöffnung.

Der Gelenkeinsatz 20 in den Figuren 3a und 3b ist im Bereich der konkaven Gelenkfläche 21 nicht nur als Nussgelenk ausgeführt, sondern weist in dem über den Äquator hinausgehenden Bereich 25 hin zur Öffnung der konkaven Gelenkfläche 21 zudem eine zusätzliche Sicherungseinrichtung in Form einer Aussparung 26 auf. Diese ist für das Einsetzen eines Sicherungsrings vorgesehen. Folglich ist bei dem gezeigten Ausführungsbeispiel der Gelenkeinsatz 20 auf der Seite des zweiten Teilgelenks mit zwei Sicherungseinrichtungen ausgeführt, die einer Trennung der konvexen Gelenkfläche 32 des Gelenkkopfes 30 von der konkaven Gelenkfläche 21 des Gelenkeinsatzes 20 vorbeugen. Allerdings ist es genauso möglich, nur eine der Sicherungseinrichtungen 25, 26 vorzusehen, um eine Luxation des jeweiligen Teilgelenks zu verhindern.

Als Sicherungsring wird bevorzugt ein länglicher Streifen aus einem flexiblen Material verwendet. Vorzugsweise ist der längliche Streifen C-förmig vorgeformt. Zum Einführen des Sicherungsrings in die umlaufende Aussparung 26, wird er durch eine Zugangsöffnung oder Zugangsaussparung, die vom Rand einer konkaven Gelenkfläche 11, 21 zu der Aussparung 26 verläuft, der Länge nach in die Aussparung 26 hineingeschoben. Ist der flexible C-förmige Sicherungsring nicht vorgeformt, wird dieser erst durch das Einführen in die umlaufende Aussparung 26 C-förmig. Alternativ ist es auch möglich, einen Sprengring als Sicherungsring zu verwenden.

Weiterhin weist der in den Figuren 3a und 3b gezeigte Gelenkeinsatz 20 auf der Seite des ersten Teilgelenks, d.h. auf der konvexen Fläche 22 des Gelenkeinsatzes 20 eine Einsetzhilfe 27 in Form einer länglichen Nut auf. Diese Nut 27 verläuft von der umlaufenden Kante 24 der konvexen Gelenkfläche 22 über den Pol 29 wieder zu der umlaufenden Kante 24. Die Berührungspunkte der Nut 27 mit der umlaufenden Kante 24 liegen sich im gezeigten Ausführungsbeispiel diametral gegenüber, da sich die Einsetzhilfe 27 in einer Symmetrieebene der kugelförmigen konvexen Gelenkfläche 22 befindet.

Die in den Figuren 4a und 4b gezeigte Gelenkpfanne 10, welche die Aufnahme für das erste Teilgelenk ausbildet, weist eine kugelsegmentförmige konkave Gelenkfläche 11 auf sowie eine für die Implantation in das Knochengewebe eines Patienten vorgesehene Außenfläche 17. Die Außenfläche 17 kann über verschiedene aus dem Stand der Technik bekannte Techniken in dem Knochengewebe verankert werden. Wie oben bereits beschrieben, weist die Gelenkpfanne 10 wie auch der Gelenkeinsatz 20 einen Bereich 12 auf, der als Sicherungseinrichtung eine Luxation des ersten Teilgelenk, d.h. ein Herausspringen des Gelenkeinsatzes 20, verhindert.

Weiterhin sind in dem Randbereich, der zwischen der Außenfläche 17 und der konkaven Gelenkfläche 11 der Gelenkpfanne 10 ausgebildet ist, Befestigungsmittel 18 vorgesehen. Bei den Befestigungsmitteln 18 handelt es sich bei dem vorliegenden Ausführungsbeispiel um ein Gewindeloch und zwei Arretierungslöcher, mit Hilfe derer die Gelenkblockiereinrichtung 13 aus Figur 2 über ein Verbindungselement 14 lösbar befestigt wird. Wie den Figuren 2 und 4 zu entnehmen ist, befindet sich das Gewindeloch 18 der Gelenkpfanne 10 dabei auf dem Umfang des Randbereichs jeweils um 90° versetzt zwischen den zwei diametral gegenüberliegenden Arretierungslöchern.

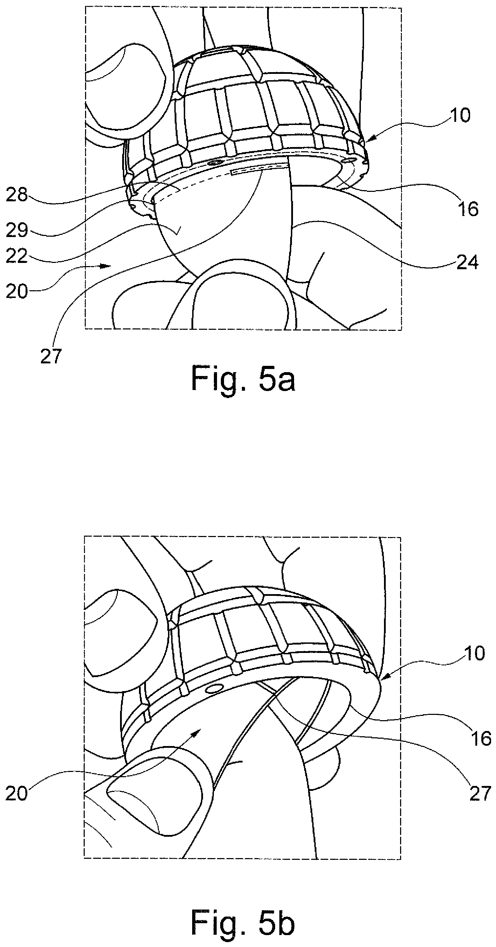

Nachfolgend wird anhand der Figuren 5a und 5b erläutert, wie eine Einsetzhilfe 27 das Einsetzen eines Gelenkeinsatzes 20 in eine Gelenkpfanne 10 unterstützt. Allerdings lässt sich das Folgende wie auch das Vorhergehende ohne weiteres auch auf das Teilgelenk übertragen, das von der konkaven Gelenkfläche 21 des Gelenkeinsatzes 20 und der konvexen Gelenkfläche 32 des Gelenkkopfes 30 gebildet wird.

Die Figuren 5a und 5b zeigen einen erfindungsgemäßen Gelenkeinsatz 20 während des Einsetzens in eine Gelenkpfanne 10. Bei dem vorliegenden Ausführungsbeispiel sind die Gelenkpfanne 10 und der Gelenkeinsatz 20 Komponenten einer Double Mobility Hüftgelenkendoprothese. Die Gelenkpfanne 10 weist eine innere konkave Gelenkfläche 11 auf, die mit der konvexen Gelenkfläche 22 des Gelenkeinsatzes 20 zusammenwirkt. Die Öffnung der konkaven Gelenkfläche 11 der Gelenkpfanne 10 ist durch eine umlaufende Kante 16 begrenzt und weist einen Durchmesser auf, der kleiner ist als der Durchmesser der konkaven Gelenkfläche 11 auf der Höhe des Äquators der Gelenkpfanne 10.

Wie weiterhin in den Figuren 5a und 5b zu erkennen ist, weist die konvexe Gelenkfläche 22 des Einsatzes 20 eine Einsetzhilfe 27 auf, die sich in einer Symmetrieebene des Einsatzes 20 bzw. der konvexen Gelenkfläche 22 befindet. Die gezeigte Einsetzhilfe 27 ist als längliche Aussparung ausgeführt, die sich von der umlaufenden Kante 24 in Richtung des Pols 29 der konvexen Gelenkfläche 22 erstreckt. Die längliche Aussparung 27 liegt in der durch die Hilfslinie 28 angedeuteten Symmetrieebene der konvexen Gelenkfläche 22.

Dadurch, dass sich die Einsetzhilfe 27 bis an die umlaufende Kante 24 der konvexen Fläche 22 erstreckt, ist es möglich, den Transport von Gelenkflüssigkeit zwischen der konvexen Fläche 22 des Einsatzes 27 und der konkaven Fläche 11 der Pfanne 10 als auch den Abtransport möglicher Abriebprodukte zwischen diesen Flächen zu fördern und somit die Lebensdauer der Gelenkendoprothese zu erhöhen.

Die längliche Aussparung beginnt an der umlaufenden Kante 24 der konvexen Fläche 22 und erstreckt sich in den Figuren 5a und 5b über ungefähr ein Drittel der Länge der Hilfslinie 28 zwischen dem Pol 29 und der Kante 24. Die längliche Aussparung erstreckt sich über 25 % bis 75 % der Verbindungslinienlänge.

Es versteht sich, dass die längliche Aussparung nicht zwangsläufig mit der umlaufenden Kante 24 der konvexen Fläche 22 in Berührung sein muss, sondern ebenso mit beiden Enden in der konvexen Fläche 22 des Gelenkeinsatzes 20 angeordnet sein kann. Weiterhin kann die Einsetzhilfe 27 mehrteilig ausgeführt sein. So ist es möglich symmetrisch zu der in Fig. 5a gezeigten Einsetzhilfe 27 einen weiteren Teil der Einsetzhilfe 27 auf der gegenüberliegenden Seite der Gelenkfläche 22 vorzusehen.

Je länger und breiter die Einsetzhilfe 27 ausgeführt wird, desto einfacher kann der Gelenkeinsatz 20 in eine als Nussgelenk ausgeführte Gelenkpfanne 10 eingesetzt werden. Es ist jedoch zu beachten, dass bei ansteigender Länge bzw. Breite die konvexe Fläche des Einsatzes unregelmäßiger wird, was ein lokales Ansteigen von Spannungen mit sich ziehen kann.

Die Form der Aussparung ist vorzugsweise korrespondierend zu der Kontur der Kante der zugehörigen konkaven Gelenkfläche, die als Gelenkpartner des Teilgelenks dient, ausgeführt. Dabei kommt die Aussparung der Einsetzhilfe 27 mit ihrem tiefsten Abschnitt beim Anlegen an der Gelenkpfanne 10 bevorzugt zumindest über einen Abschnitt ihres Aussparungsbodens mit der umlaufenden Kante 16 der konkaven Aufnahme eines Gelenkpartners, hier der Gelenkpfanne 10, in Berührung. Am bevorzugtesten weist die Aussparung 27 in ihrer Längsrichtung ein Profil mit einem Radius auf, der im Wesentlichen dem Radius R_{Pi} der Gelenkpfanne 10 bei ihrer umlaufenden Kante 16 gleicht. Dabei geht die Einsetzhilfe 27 bevorzugt zumindest an einem ihrer Enden in Längsrichtung graduell in die konvexe Fläche des Einsatzes über.

Der Querschnitt der länglichen Aussparung ist so ausgeführt, um das Entstehen von Spannungsspitzen im Bereich des Übergangs von der Einsetzhilfe 27 zu der Gelenkfläche 22 zu vermeiden. Aus diesem Grund geht die Einsetzhilfe bevorzugt fließend in die konvexe Gelenkfläche 22 des Gelenkeinsatzes 20 über.

Bei der gezeigten Ausführungsform ist die aussparungsförmige Einsetzhilfe 27 auf der Hilfslinie 28 zwischen dem Rand der konvexen Gelenkfläche 22 und deren Pol 29 angeordnet. Mit anderen Worten bildet die durch die Aussparung ausgebildete Ebene mit der durch die Hilfslinie 28 aufgespannten Ebene einen Winkel von 0°. Auch wenn dies die bevorzugte Ausführungsform ist, so ist es ohne weiteres möglich, einen beliebigen anderen Winkel zu wählen.

Mit anderen Worten kann der Winkel zwischen der Einsetzhilfe 27 und der Hilfslinie 28 zwischen 0° bis 90° betragen, jedoch bevorzugt 0° bis 45° und noch bevorzugter 0° bis 15°. Im Allgemeinen gilt der Grundsatz, dass je kleiner der Winkel zwischen der Einsetzhilfe 27 und der gedachten Hilfslinie 28, desto geringer ist die Wahrscheinlichkeit, dass sich die Einsetzhilfe 27 während der auftretenden Bewegung des Gelenks in Flucht mit der Öffnungskante der Gelenkpfanne begibt, sodass es zu einer Luxation kommen kann.

Alternativ ist es möglich, die durch die Einsetzhilfe 27 aufgespannte Ebene parallel zu der durch die Hilfslinie 28 aufgespannten Ebene bzw. der Symmetrieebene der konvexen Gelenkfläche 22 zu versetzen.

Eine derart freie Positionierung der Einsetzhilfe 27 auf der konvexen Fläche 22 des Einsatzes 20 ermöglicht eine Optimierung hinsichtlich einer einfachen Montage des Gelenkeinsatzes 20 in einer Gelenkpfanne 10 oder eines Gelenkkopfes 30 in einem Gelenkeinsatz 20. So kann die trotz der Einsetzhilfe 27 noch hervorgerufene Belastung des Implantatmaterials bei der Montage des künstlichen Gelenks berücksichtigt werden. Auch ist es möglich, eine Materialbelastung der Gelenkpfanne 10, des Einsatzes 20 und/oder des Gelenkkopfes 30 in Abhängigkeit vom Implantationsort und den sich daraus ergebenden Belastungen zu reduzieren. Somit ist es durch die Einsetzhilfe 27 möglich, die Gelenkpfanne 10 und insbesondere den Gelenkeinsatz 20 bzw. den Gelenkkopf 30 aus einem relativ unelastischen Material herzustellen, wie zum Beispiel Metall oder Keramik.

Da bei der gezeigten Ausführungsform die Einsetzhilfe 27 auf der Hilfslinie 28, d. h. in einer Symmetrieebene des Gelenkeinsatzes 20, und damit in einem Winkel von 0° angeordnet ist, erfolgt das Einsetzen des Gelenkeinsatzes 20 in die Gelenkpfanne 10, indem die beiden Komponenten vor dem Einsetzen um in etwa 90° zueinander geneigt werden. Ist die Einsetzhilfe 27 in einem anderen Winkel angeordnet, so müssen die Gelenkpfanne 10 und der Gelenkeinsatz 20 für die Montage entsprechend anders zueinander ausgerichtet werden.

Nach dem Ausrichten wird die Einsetzhilfe 27 auf die umlaufende Kante 26 der konkaven Gelenkpfannenfläche 11 gesetzt, sodass die umlaufende Kante 16 innerhalb der Aussparung der Einsetzhilfe 27 angeordnet ist. Auf diese Weise befindet sich der Einsatz 20 in der Ebene der Öffnung der konkaven Gelenkfläche 11 in einer relativ zum montierten Zustand leicht versetzten Position und passt so durch die von der konkaven Gelenkfläche 11 ausgebildeten Öffnung. Anders ausgedrückt gleicht der Versatz das Übermaß des Gelenkeinsatzes 20 im Verhältnis zu der Öffnung der Gelenkpfanne 10 aus.

Mit anderen Worten passt der Gelenkeinsatz 20 in die konkave Gelenkfläche 11 der Gelenkpfanne 10, da der Durchmesser des Gelenkeinsatzes 20 auf der Höhe der umlaufenden Kante 24 der konvexen Fläche 22 des Gelenkeinsatzes 20 aufgrund der Tiefe der Einsetzhilfe 27 kleiner oder gleich dem Durchmesser der umlaufenden Kante 16 der konkaven Gelenkfläche 11 ist.

In Figur 5b wird der Gelenkeinsatz 20 nach erfolgreichem Einsetzen in die Gelenkpfanne 10 in leichter Schrägstellung gezeigt. Weiterhin ist zu erkennen, dass der Gelenkeinsatz 20 dieses Ausführungsbeispiels eine konkave Gelenkfläche 21 aufweist, die für die Aufnahme eines Gelenkkopfes 30 (siehe Figur 2) vorgesehen ist.

In jedem Fall wird durch die vorliegende Erfindung eine luxationsfreie Gelenkendoprothese bereitgestellt, die dennoch einen einfachen Zusammenbau während eines operativen Eingriffs ermöglicht.

### BEZUGSZEICHEN

- 1: Hüftpfanne (Stand der Technik)
- 2: Gelenkeinsatz (Stand der Technik)
- 3: Gelenkkopf (Stand der Technik)
- 4: Gelenkschaft (Stand der Technik)
- 10: Gelenkpfanne
- 11: konkave Gelenkfläche der Gelenkpfanne
- 12: Sicherungseinrichtung der Gelenkpfanne: Vorsprung
- 13: Gelenkblockiereinrichtung
- 14: Verbindungselement
- 15: umlaufender Rand der Gelenkpfanne
- 16: umlaufende Kante der Gelenkpfanne
- 17: Außenfläche der Gelenkpfanne
- 18: Befestigungsmittel
- 20: Gelenkeinsatz
- 21: konkave Gelenkfläche des Gelenkeinsatzes
- 22: konvexe Gelenkfläche des Gelenkeinsatzes
- 23: umlaufende Kante der konkaven Gelenkfläche des Gelenkeinsatzes
- 24: umlaufende Kante der konvexen Gelenkfläche des Gelenkeinsatzes
- 25: Sicherungseinrichtung des Gelenkeinsatzes: Vorsprung
- 26: Sicherungseinrichtung des Gelenkeinsatzes: Aussparung für Sicherungsring
- 27: Einsetzhilfe: Aussparung
- 28: Hilfslinie auf der konvexen Gelenkfläche des Gelenkeinsatzes 20, die in dessen Symmetrieebene verläuft
- 29: Pol der konvexen Gelenkfläche 22
- 30: Gelenkkopf
- 32: konvexe Gelenkfläche des Gelenkkopfes
- 40: Prothesenschaft
- 50: Drückwerkzeug
- Aq: Äquator einer konvexen bzw. konkaven Gelenkfläche
- R_{Pi}: innerer Radius der Gelenkpfanne
- D_{Pi}: Gelenkinnendurchmesser der Gelenkpfanne
- D_{Po}: Gelenköffnungsdurchmesser der Gelenkpfanne
- R_{Ei}: innerer Radius des Gelenkeinsatzes
- D_{Eo}: Gelenkinnendurchmesser des Gelenkeinsatzes
- D_{Ei}: Gelenköffnungsdurchmesser des Gelenkeinsatzes

## Patentansprüche

1. Gelenkersatz mit
einer Gelenkpfanne (10), die eine konkave Gelenkfläche (11) aufweist, einem Gelenkkopf (30), der eine konvexe Gelenkfläche (32) aufweist, die durch eine umlaufende Kante begrenzt ist, und
einem Gelenkeinsatz (20), der eine konkave (21) und eine konvexe Gelenkfläche (22) aufweist, die jeweils durch eine umlaufende Kante (23, 24) begrenzt sind,
wobei die konvexe Gelenkfläche (22) des Gelenkeinsatzes (20) ausgelegt ist, um im montierten Zustand mit der konkaven Gelenkfläche (11) der Gelenkpfanne (10) ein erstes Teilgelenk auszubilden,
die konkave Gelenkfläche (21) des Gelenkeinsatzes (20) ausgelegt ist, um im montierten Zustand mit der konvexen Gelenkfläche (32) des Gelenkkopfes (30) ein zweites Teilgelenk auszubilden und
die Gelenkpfanne (10) und der Gelenkeinsatz (20) jeweils eine Sicherungseinrichtung (12, 25, 26) aufweisen, die einer Luxation des Gelenkersatzes vorbeugt,
wobei der Gelenkkopf (30) und/oder der Gelenkeinsatz (20) eine Einsetzhilfe (27, 28) aufweist, mit der das Einsetzen der jeweiligen konvexen Gelenkfläche (22, 32) in die dazugehörige konkave Gelenkfläche (11, 21) ermöglicht wird,
**dadurch gekennzeichnet, dass** die Einsetzhilfe (27) als eine längliche Aussparung in der konvexen Gelenkfläche (22, 32) von zumindest einem der Teilgelenke ausgebildet ist, wobei die längliche Aussparung an der umlaufenden Kante (24) der konvexen Gelenkfläche (22, 32) beginnt und sich über 25 % bis 75 % der Länge einer Hilfslinie (28) erstreckt, die auf der konvexen Gelenkfläche (22, 32) in dessen Symmetrieebene zwischen der umlaufenden Kante (24) und einem Pol (29) der Gelenkfläche (22, 32) verläuft.

2. Gelenkersatz nach Anspruch 1,
bei dem ein Bereich (12, 25) der konkaven Gelenkfläche (11, 21) der Gelenkpfanne (10) und/oder des Gelenkeinsatzes (20) der Luxation des jeweiligen Teilgelenks durch Ausbilden des jeweiligen Gelenks als Nussgelenk vorbeugt.

3. Gelenkersatz nach Anspruch 2,
bei dem der Öffnungsdurchmesser (D_{Po}, D_{Eo}) der konkaven Gelenkfläche (11, 21) 1% bis 6%, bevorzugt 2% bis 5%, am bevorzugtesten 3,5% bis 5% geringer ist als der Gelenkdurchmesser (D_{Pi},D_{Ei}).

4. Gelenkersatz nach Anspruch 2 oder 3,
bei dem die Sicherungseinrichtung (12, 25, 26) von zumindest einem der Teilgelenke zwei bevorzugt lösbare Sicherungselemente aufweist, die zusammenwirkend einer Luxation des Teilgelenks vorbeugen.

5. Gelenkersatz nach Anspruch 4,
bei dem die zwei Sicherungselemente mittels einer bevorzugt lösbaren Schraub-, Verriegelungs- und/oder Schnappverbindung zusammenwirken.

6. Gelenkersatz nach einem der Ansprüche 4 oder 5,
bei dem ein Sicherungselement durch eine bevorzugt umlaufende Aussparung (26) zur Aufnahme eines Sicherungsrings in der konkaven Gelenkfläche (11, 21) der Gelenkpfanne (10) und/oder des Gelenkeinsatzes (20) ausgebildet ist.

7. Gelenkersatz nach einem der Ansprüche 4 bis 6,
bei dem ein Sicherungselement durch mindestens eine Aussparung in der Gelenkpfanne (10) und/oder in dem Gelenkeinsatz (20) ausgebildet ist, welches mit einem Vorsprung eines weiteren Sicherungselements eine Bajonettverbindung ausbildet.

8. Gelenkersatz nach einem der vorstehenden Ansprüche,
bei dem die Einsetzhilfe (27) in der Symmetrieebene der konvexen Gelenkfläche (22, 32) ausgebildet ist.

## Claims

1. joint replacement comprising a joint socket (10) having a concave joint surface (11), a joint head (30) having a convex joint surface which is delimited by a peripheral edge, and a joint insert (20) which has a concave (21) and a convex joint surface (22), which are each bounded by a peripheral edge (23, 24),
wherein the convex joint surface (22) of the joint insert (20) is designed to form a first partial joint with the concave joint surface (11) of the joint socket (10) in the assembled state, wherein the concave joint surface (21) of the joint insert (20) is designed to form a second partial joint with the convex joint surface (32) of the joint head (30) in the mounted state, and the joint socket (10) and the joint insert (20) each have a securing device (12, 25, 26) which prevents dislocation of the joint replacement,
wherein the joint head (30) and/or the joint insert (20) has an insertion aid (27, 28) which enables the insertion of the respective convex joint surface (22, 32) into the associated concave joint surface (11, 21),
**characterised in that** the insertion aid (27) is configured as an elongated recess in the convex joint surface (22, 32) of at least one of the partial joints, wherein the elongated recess begins at the peripheral edge (24) of the convex joint surface (22, 32) and extends over 25% to 75% of the length of an auxiliary line (28) running on the convex joint surface (22, 32) in its plane of symmetry between the peripheral edge (24) and a pole (29) of the joint surface (22, 32).

2. The joint replacement according to claim 1,
in which a region (12, 25) of the concave joint surface (11, 21) of the joint socket (10) and/or of the joint insert (20) prevents dislocation of the respective partial joint by forming the respective joint as a ball-and-socket joint.

3. The joint replacement according to claim 2,
in which the opening diameter (D_{Po}, D_{Eo}) of the concave joint surface (11, 21) is 1% to 6%, preferably 2% to 5%, most preferably 3.5% to 5% smaller than the joint diameter (D_{Pi}, D_{Ei}).

4. The joint replacement according to claim 2 or 3,
in which the securing device (12, 25, 26) of at least one of the partial joints has two preferably releasable securing elements which cooperate to prevent dislocation of the partial joint.

5. The joint replacement according to claim 4,
in which the two securing elements cooperate by means of a preferably releasable screw, locking, and/or snap connection.

6. The joint replacement according to any one of claims 4 or 5, in which a securing element is formed by a preferably circumferential recess (26) for receiving a securing ring in the concave joint surface (11, 21) of the joint socket (10) and/or the joint insert (20).

7. The joint replacement according to any one of claims 4 to 6, in which a securing element which forms a bayonet connection with a projection of another securing element is formed by at least one recess in the joint socket (10) and/or in the joint insert (20).

8. The joint replacement according to any one of the preceding claims,
in which the insertion aid (27) is formed in the plane of symmetry of the convex joint surface (22, 32).

## Revendications

1. Prothèse articulaire comportant une cavité articulaire (10) présentant une surface articulaire concave (11), une tête articulaire (30) présentant une surface articulaire convexe (32) délimitée par un bord périphérique, et un insert articulaire (20) présentant une surface articulaire concave (21) et une surface articulaire convexe (22), délimitées chacune par un bord périphérique (23, 24),
dans laquelle la surface articulaire convexe (22) de l'insert articulaire (20) est conçue pour former une première articulation partielle avec la surface articulaire concave (11) de la cavité articulaire (10) à l'état assemblé,
la surface articulaire concave (21) de l'insert articulaire (20) est conçue pour former une seconde articulation partielle avec la surface articulaire convexe (32) de la tête articulaire (30) à l'état assemblé et
la cavité articulaire (10) et l'insert articulaire (20) présentent chacun un dispositif de fixation (12, 25, 26) qui empêche une luxation de la prothèse articulaire,
dans laquelle la tête articulaire (30) et/ou l'insert articulaire (20) présentent un dispositif d'aide à l'insertion (27, 28) avec lequel l'insertion de la surface articulaire convexe respective (22, 32) dans la surface articulaire concave (11, 21) associée est possible,
**caractérisée en ce que** le dispositif d'aide à l'insertion (27) est réalisé sous la forme d'un évidement allongé dans la surface articulaire convexe (22, 32) d'au moins l'une des articulations partielles, dans lequel l'évidement allongé commence au niveau du bord périphérique (24) de la surface articulaire convexe (22, 32) et s'étend sur 25 % à 75 % de la longueur d'une ligne auxiliaire (28) qui s'étend sur la surface articulaire convexe (22, 32) dans son plan de symétrie entre le bord périphérique (24) et un pôle (29) de la surface articulaire (22, 32).

2. Prothèse articulaire selon la revendication 1,
dans laquelle une région (12, 25) de la surface articulaire concave (11, 21) de la cavité articulaire (10) et/ou de l'insert articulaire (20) empêche la luxation de l'articulation partielle respective en formant l'articulation respective sous la forme d'une articulation à écrou.

3. Prothèse articulaire selon la revendication 2,
dans laquelle le diamètre d'ouverture (D_{Po}, D_{Eo}) de la surface articulaire concave (11, 21) est de 1 % à 6 %, de préférence de 2 % à 5 %, de manière préférée entre toutes de 3,5 % à 5 % inférieur au diamètre articulaire (D_{Pi}, D_{Ei}).

4. Prothèse articulaire selon la revendication 2 ou 3,
dans laquelle le dispositif de fixation (12, 25, 26) d'au moins l'une des articulations partielles présente deux éléments de fixation de préférence libérables qui coopèrent pour empêcher une dislocation de l'articulation partielle.

5. Prothèse articulaire selon la revendication 4,
dans laquelle les deux éléments de fixation interagissent au moyen d'une liaison à vis, à verrouillage et/ou à encliquetage de préférence amovible.

6. Prothèse articulaire selon l'une des revendications 4 ou 5, dans laquelle un élément de fixation est formé par un évidement (26) de préférence circonférentiel destiné à recevoir une bague de fixation dans la surface articulaire concave (11, 21) de la cavité articulaire (10) et/ou de l'insert articulaire (20).

7. Prothèse articulaire selon l'une des revendications 4 à 6, dans laquelle un élément de fixation est formé par au moins un évidement dans la cavité articulaire (10) et/ou dans l'insert articulaire (20), lequel élément forme une liaison à baïonnette avec une saillie d'un autre élément de fixation.

8. Prothèse articulaire selon l'une des revendications précédentes,
dans laquelle le dispositif d'aide à l'insertion (27) est formée dans le plan de symétrie de la surface articulaire convexe (22, 32).
